# EUROPEAN PATENT SPECIFICATION

(11) **EP 3 187 177 B1**
(45) Date of publication and mention of the grant of the patent: **20.03.2019**
(21) Application number: 16198417.4
(22) Date of filing: 07.01.2004
(51) Int. Cl.: A61K 9/22, A61K 9/20

(54) **BIODEGRADABLE OCULAR IMPLANT**
BIOLOGISCH ABBAUBARES AUGENIMPLANTAT
IMPLANT OCULAIRE BIODÉGRADABLE

(30) Priority: 09.01.2003 US 340237
(43) Date of publication of application: 05.07.2017
(62) Divisional of application: 08002892.1
(73) Proprietor: ALLERGAN, INC., Irvine, CA 92612 (US)
(72) Inventor: WEBER, David, Danville, CA 94506-6004 (US); CHOU, David, Palo Alto, CA 94306 (US); PENG, Lin, Palo Alto, CA 94303 (US); NIVAGGIOLI, Thierry, Lost Altos Hills, CA 94022 (US)
(74) Representative: Hoffmann Eitle

(56) References cited:
- WO-A-96/38174
- WO-A2-02/43785
- US-B1- 6 306 426

## Description

### FIELD OF THE INVENTION

The present invention relates to the field of ophthalmology. In particular, biodegradable implants for use in the treatment of medical conditions of the eye are provided.

### BACKGROUND OF THE INVENTION

Immunosuppressive agents are routinely used for the treatment of uveitis of various etiologies. For example, topical or oral glucocorticoids are often included in the therapeutic regimen; however, a major problem with these routes of administration is the inability to achieve an adequate intraocular drug concentration of the glucocorticoid. In fact, the difficulties of treating uveitis due to poor intraocular penetration of topical medications into the posterior segment is well known (Bloch-Michel E. (1992). "Opening address: intermediate uveitis," In Intermediate Uveitis, Dev. Ophthalmol. W.R.F. Boke et al. eds., Basel: Karger, 23:1-2; Pinar, V. Intermediate uveitis. Massachusetts Eye & Ear Infirmary Immunology Service at <http://wwwimmunology.meei.harvard.edu/imed.htm> (visited in 1998); Rao, N.A. et al. (1997). "Intraocular inflammation and uveitis," In Basic and Clinical Science Course. Section 9 (1997-1998) San Francisco: American Academy of Ophthalmology, pp. 57-80, 102-103, 152-156; Böke, W. (1992). "Clinical picture of intermediate uveitis," In Intermediate Uveitis, Dev. Ophthalmol. W.R.F. Böke et al. eds., Basel: Karger, 23:20-7; and Cheng C-K et al. (1995). "Intravitreal sustained-release dexamethasone device in the treatment of experimental uveitis," Invest. Ophthalmol. Vis. Sci. 36:442-53).

Systemic glucocorticoid administration may be used alone or in addition to topical glucocorticoids for the treatment of uveitis. Prolonged exposure to high plasma concentrations (administration of 1 mg/kg/day for 2-3 weeks) of steroid is often necessary so that therapeutic levels can be achieved in the eye (Pinar, V. "Intermediate uveitis," Massachusetts Eye & Ear Infirmary Immunology Service at <http://www.immunology.meei.harvard.edu/imed.htrn> (visited in 1998)).

However, these high drug plasma levels commonly lead to systemic side effects such as hypertension, hyperglycemia, increased susceptibility to infection, peptic ulcers, psychosis, and other complications (Cheng C-K et al. (1995). "Intravitreal sustained-release dexamethasone device in the treatment of experimental uveitis," Invest. Ophthalmol. Vis. Sci. 36:442-53; Schwartz, B. (1966). "The response of ocular pressure to corticosteroids," Ophthalmol. Clin. North Am. 6:929-89; Skalka, H.W. et al. (1980). "Effect of corticosteroids on cataract formation," Arch Ophthalmol 98:1773-7; and Renfro, L. et al. (1992). "Ocular effects of topical and systemic steroids," Dermatologic Clinics 10:505-12).

In addition, overall drug delivery to the eye may be poor for drugs with short plasma half-lives since their exposure to intraocular tissues is limited. Therefore, the most efficient way of delivering a drug to the posterior segment is to place it directly into the vitreous (Maurice, D.M. (1983). "Micropharmaceutics of the-eye," Ocular Inflammation Ther. 1:97-102; Lee, V.H.L. et al. (1989). "Drug delivery to the posterior segment" Chapter 25 In Retina. T.E. Ogden and A.P. Schachat eds., St. Louis: CV Mosby, Vol. 1, pp. 483-98; and Olsen, T.W. et al. (1995). "Human scleral permeability: effects of age, cryotherapy, transscleral diode laser, and surgical thinning," Invest. Ophthalmol. Vis. Sci. 36:1893-1903).

Techniques such as intravitreal injection have shown promising results, but due to the short intraocular half-life of glucocorticoids (approximately 3 hours), intravitreal injections must be repeated to maintain drug levels. In turn, this repetitive process increases the potential for side effects such as retinal detachment, endophthalmitis, and cataracts (Maurice, D.M. (1983). "Micropharmaceutics of the eye," Ocular Inflammation Ther. 1:97-102; Olsen, T.W. et al. (1995). "Human scleral permeability: effects of age, cryotherapy, transscleral diode laser, and surgical thinning," Invest. Ophthalmol. Vis. Sci. 36:1893-1903; and Kwak, H.W. and D'Amico, D. J. (1992). "Evaluation of the retinal toxicity and pharmacokinetics of dexamethasone after intravitreal injection," Arch. Ophthalmol. 110:259-66).

One of the alternatives to intravitreal injection to administer drugs is the placement of biodegradable implants under the sclera or into the subconjunctival or suprachoroidal space, as described in U.S. 4,863,457 to Lee; WO 95/13765 to Wong et al.; WO 00/37056 to Wong et al.; EP 430,539 to Wong; in Gould et al., Can. J. Ophthalmol. 29(4):168-171 (1994); and in Apel et al., Curr. Eye Res. 14:659-667 (1995).

Furthermore, .the controlled release of drugs from polylactide/polyglycolide (PLGA) copolymers into the vitreous has been disclosed, e.g., in U.S. 5,501,856 to Ohtori et al. and EP 654,256 to Ogura. Also WO96/38174 discloses an ocular implant obtained by extrusion for use in the human eye comprising 50% of dexamethasone and 50% of PLGA.

Recent experimental work has demonstrated that uncapped PLGA degrades faster than capped (end-capped) PLGA (Park et al., J. Control. Rel. 55:181-191 (1998); Tracy et al., Biomaterials 20:1057-1062 (1999); and Jong et al., Polymer 42:2795-2802 (2001). Accordingly, implants containing mixtures of uncapped and capped PLGA have been formed to modulate drug release. For example, U.S. 6,217,911 to Vaughn et al. ('911) and U.S. 6,309,669 to Setterstrom et al. ('669) disclose the delivery of drugs from a blend of uncapped and capped PLGA copolymer to curtail initial burst release of the drugs. In the '911 patent, the composition delivers non-steroidal anti-inflammatory drugs from PLGA microspheres made by a solvent extraction process or PLGA microcapsules prepared by a solvent evaporation process over a duration of 24 hours to 2 months. In the '669 patent, the composition delivers various pharmaceuticals from PLGA microcapsules over a duration of 1-100 days. The PLGA microspheres or microcapsules are administered orally or as an aqueous injectable formulation. As mentioned above, there is poor partitioning of drug into the eye with oral administration. Furthermore, use of an aqueous injectable drug composition (for injecting into the eye) should be avoided since the eye is a closed space (limited volume) with intraocular pressure ranges that are strictly maintained. Administration of an injectable may increase intraocular volume to a point where intraocular pressures would then become pathologic.

Consequently, a biodegradable implant for delivering a therapeutic agent to an ocular region may provide significant medical benefit for patients afflicted with a medical condition of the eye.

The scope of the invention is defined by the claims. Any references in the description to methods of treatment refer to the compounds, pharmaceutical compositions and medicaments of the present invention for use in a treatment of the human(or animal)body by therapy (or diagnosis).

### SUMMARY OF THE INVENTION

The biodegradable implants claimed are used for the treatment of medical conditions of the eye. Consequently, the claimed implants are sized such that they are appropriate for implantation in the intended ocular region.

The bioerodible implant for treating medical conditions of the eye includes an active agent dispersed within a biodegradable polymer matrix, wherein the bioerodible implant has an *in vivo* in rabbit eye cumulative release profile in which less than about 15 percent of the active agent is released about one day after implantation of the bioerodible implant and greater than about 80 percent of the active agent is released about 28 days after implantation of the bioerodible implant, and wherein the biodegradable polymer matrix comprises a mixture of hydrophilic end group PLGA and hydrophobic end group PLGA.

The bioerodible implant for treating medical conditions of the eye includes an active agent dispersed within a biodegradable polymer matrix, wherein the bioerodible implant is formed by an extrusion method, wherein the extrusion method comprises using a piston extruder, a single screw extruder or a twin screw extruder, and wherein the bioerodible implant has an *in vivo* in rabbit eye cumulative release profile in which greater than about 80 percent of the active agent is released about 28 days after implantation of the bioerodible implant.

In a further variation, the bioerodible implant for treating medical conditions of the eye includes an active agent dispersed within a biodegradable polymer matrix, wherein the bioerodible implant exhibits a cumulative release profile in which greater than about 80 percent of the active agent is released about 28 days after implantation of the bioerodible implant, and wherein the cumulative release profile is approximately sigmoidal in shape over about 28 days after implantation.

The claimed bioerodible implant for treating medical conditions of the eye includes an active agent dispersed within a biodegradable polymer matrix, wherein the biodegradable polymer matrix comprises a mixture of PLGA having hydrophilic end groups and PLGA having hydrophobic end groups. The hydrophilic end groups include, carboxyl, hydroxyl, and polyethylene glycol. The hydrophobic end groups include, alkyl esters and aromatic esters.

In yet another variation, the bioerodible implant for treating medical conditions of the eye includes an active agent dispersed within a biodegradable polymer matrix, wherein the bioerodible implant has an *in vivo* in rabbit eye cumulative release profile in which less than about 15 percent of the active agent is released about one day after implantation of the bioerodible implant and greater than about 80 percent of the active agent is released about 28 days after implantation of the bioerodible implant.

Various active agents may be incorporated into the bioerodible implants. In one variation, anti-inflammatory agents, including, but not limited to nonsteroidal anti-inflammatory agents and steroidal anti-inflammatory agents may be used. In another variation, active agents that may be used in the bioerodible implants are ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, antibacterials, antihypertensives, pressors, antiprotozoal agents, antiviral agents, antifungal agents, anti-infective agents, antitumor agents, antimetabolites, and antiangiogenic agents.

The implants defined in the claims are used to treat medical conditions of the eye in mammalian subjects, e.g., human subjects. Examples of such medical conditions include, but are not limited to, uveitis, macular edema, macular degeneration, retinal detachment, ocular tumors, fungal or viral infections, multifocal choroiditis, diabetic retinopathy, proliferative vitreoretinopathy (PVR), sympathetic opthalmia, Vogt Koyanagi-Harada (VKH) syndrome, histoplasmosis, uveal diffusion, vascular occlusion, and the like.

Furthermore, upon implantation in an ocular region of the subject, the bioerodible implants deliver the active agent such that the resulting concentration of active agent *in vivo* in rabbit aqueous humor is approximately 10-fold less than in rabbit vitreous humor. The active agent is delivered so that a therapeutic amount of active agent is provided in the ocular region of interest. In general, the therapeutic amount of active agent in an ocular region may be modified by varying the size of the bioerodible implant.

### BRIEF DESCRIPTION OF THE OF THE DRAWINGS

Figure 1 shows the *in vivo* concentration of dexamethasone in the vitreous of rabbit eyes over a 42 day period after implantation of compressed and extruded biodegradable implants containing 350 *µ*g dexamethasone into the posterior segment of rabbit eyes .
Figure 2 shows the *in vivo* cumulative percentage release of dexamethasone in the vitreous of rabbit eyes over a 42 day period after implantation of compressed and extruded biodegradable implants containing 350 *µ*g dexamethasone and 700 *µ*g dexamethasone into the posterior segment of rabbit eyes.
Figure 3 shows the *in vivo* concentration of dexamethasone in the aqueous humor of rabbit eyes over a 42 day period after implantation of compressed and extruded biodegradable implants containing 350 *µ*g dexamethasone into the posterior segment of rabbit eyes.
Figure 4 shows the *in vivo* concentration of dexamethasone in the plasma (from a rabbit blood sample) over a 42 day period after implantation of compressed and extruded biodegradable implants containing 350 *µ*g dexamethasone into the posterior segment of rabbit eyes.
Figure 5 shows the *in vivo* concentration of dexamethasone in the vitreous of rabbit eyes over a 42 day period after implantation of compressed and extruded biodegradable implants containing 700 *µ*g dexamethasone into the posterior segment of rabbit eyes.
Figure 6 shows the *in vivo* concentration of dexamethasone in the aqueous humor of rabbit eyes over a 42 day period after implantation of compressed and extruded biodegradable implants containing 700 *µ*g dexamethasone into the posterior segment of rabbit eyes.
Figure 7 shows the *in vivo* concentration of dexamethasone in the plasma (from a rabbit blood sample) over a 42 day period after implantation of compressed and extruded biodegradable implants containing 700 *µ*g dexamethasone into the posterior segment of rabbit eyes.
Figure 8 shows the *in vivo* concentration of dexamethasone in the vitreous of rabbit eyes over a 42 day period after implantation of compressed and extruded biodegradable implants containing 350 *µ*g dexamethasone and 700 *µ*g dexamethasone into the posterior segment of rabbit eyes.
Figure 9 shows the *in vitro* total cumulative percentage release of dexamethasone into a saline solution at 37°C from 60/40 w/w dexamethasone/PLGA implants having a weight ratio of 40:0 hydrophobic end to hydrophilic end PLGA (312-140-2), weight ratio of 30:10 hydrophobic end to hydrophilic end PLGA (312-140-4), weight ratio of 20:20 hydrophobic end to hydrophilic end PLGA (312-140-3), and weight ratio of 0:40 hydrophobic end to hydrophilic end PLGA (312-140-1).
Figure 10 compares the *in vitro* cumulative percentage release of dexamethasone into a saline solution at 37°C for six lots of extruded implants having 60% by weight dexamethasone, 30% by weight hydrophilic end PLGA, and 10% by weight hydrophobic end PLGA.

### DETAILED DESCRIPTION OF THE INVENTION

The present invention provides biodegradable ocular implants for use in the treatment of medical conditions of the eye. Usually, the implants are formed to be monolithic, i.e., the particles of active agent are distributed throughout the biodegradable polymer matrix. Furthermore, the implants are formed to release an active agent into an ocular region of the eye over various time periods. The active agent may be release over a time period including, but is not limited to, approximately six months, approximately three months, approximately one month, or less than one month.

### Definitions

For the purposes of this description, we use the following terms as defined in this section, unless the context of the word indicates a different meaning.

As used herein, the term "ocular region" refers generally to any area of the eyeball, including the anterior and posterior segment of the eye, and which generally includes, but is not limited to, any functional (e.g., for vision) or structural tissues found in the eyeball, or tissues or cellular layers that partly or completely line the interior or exterior of the eyeball. Specific examples of areas of the eyeball in an ocular region include the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

By "subject" it is meant mammalian subjects, preferably humans. Mammals include, but are not limited to, primates, farm animals, sport animals, e.g., horses (including race horses), cats, dogs, rabbits, mice, and rats.

As used herein, the term "treat" or "treating" or "treatment" refers to the resolution, reduction, or prevention of a medical condition of the eye or the sequelae of a medical condition of the eye.

As used herein, the terms "active agent" and "drug" are used interchangeably and refer to any substance used to treat a medical condition of the eye.

As used herein, the term "medical condition" refers to conditions that are generally treated non-invasively, e.g., with drugs, as well as conditions that are generally treated using a surgical procedure.

By "therapeutic amount" it is meant a concentration of active agent that has been locally delivered to an ocular region that is appropriate to safely treat a medical condition of the eye.

As used herein, the term "cumulative release profile" refers to the cumulative total percent of agent released from the implant either into the posterior segment *in vivo* in rabbit eyes over time or into the specific release medium *in vitro* over time.

### Biodegradable Implants For Treating Medical Conditions of the Eye

The implants of the invention include an active agent dispersed within a biodegradable polymer, wherein the biodegradable polymer matrix comprises a mixture of (i) PLGA having hydrophilic end groups, wherein said hydrophilic end group is selected from carboxyl, hydroxyl, polyethylene glycol, or a combination thereof, and (ii) PLGA having hydrophobic end groups, wherein said hydrophobic end group is an alkyl ester or aromatic ester. The implant compositions typically vary according to the preferred drug release profile, the particular active agent used, the condition being treated, and the medical history of the patient. Active agents that may be used include, but are not limited to, ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, anti-inflammatory agents, antihypertensives, pressors, antibacterials, antivirals, antifungals, antiprotozoals, antiinfectives, antitumor agents, antimetabolites, and antiangiogenic agents.

In one variation the active agent is methotrexate. In another variation, the active agent is retinoic acid. In a preferred variation, the anti-inflammatory agent is a nonsteroidal anti-inflammatory agent. Nonsteroidal anti-inflammatory agents that may be used include, but are not limited to, aspirin, diclofenac, flurbiprofen, ibuprofen, ketorolac, naproxen, and suprofen. In a more preferred variation, the anti-inflammatory agent is a steroidal anti-inflammatory agent.

### Steroidal Anti-Inflammatory Agents

The steroidal anti-inflammatory agents that may be used in the ocular implants include, but are not limited to, 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, and any of their derivatives.

In one variation, cortisone, dexamethasone, fluocinolone, hydrocortisone, methylprednisolone, prednisolone, prednisone, and triamcinolone, and their derivatives, are preferred steroidal anti-inflammatory agents. In another preferred variation, the steroidal anti-inflammatory agent is dexamethasone. In another variation, the biodegradable implant includes a combination of two or more steroidal anti-inflammatory agents.

The steroidal anti-inflammatory agent may constitute from about 10% to about 90% by weight of the implant. In one variation, the agent is from about 40% to about 80% by weight of the implant. In a preferred variation, the agent comprises about 60% by weight of the implant.

### The Biodegradable Polymer Matrix

In one variation, the active agent may be homogeneously dispersed in the biodegradable polymer matrix of the implants, wherein the biodegradable polymer matrix comprises a mixture of (i) PLGA having hydrophilic end groups, wherein said hydrophilic end group is selected from carboxyl, hydroxyl, polyethylene glycol, or a combination thereof, and (ii) PLGA having hydrophobic end groups, wherein said hydrophobic end group is an alkyl ester or aromatic ester. The biodegradable polymer matrix usually comprises at least about 10, at least about 20, at least about 30, at least about 40, at least about 50, at least about 60, at least about 70, at least about 80, or at least about 90 weight percent of the implant. In one variation, the biodegradable polymer matrix comprises about 40% by weight of the implant.

Copolymers of glycolic and lactic acid are the ones claimed, where the rate of biodegradation is controlled by the ratio of glycolic to lactic acid. The percent of each monomer in poly(lactic-co-glycolic)acid (PLGA) copolymer may be 0-100%, about 15-85%, about 25-75%, or about 35-65%. In a preferred variation, a 50/50 PLGA copolymer is used. More preferably, a random copolymer of 50/50 PLGA is used.

Biodegradable polymer matrices that include mixtures of hydrophilic and hydrophobic ended PLGA are employed, and are useful in modulating polymer matrix degradation rates. Hydrophobic ended (also referred to as capped or end-capped) PLGA has an ester linkage hydrophobic in nature at the polymer terminus. Typical hydrophobic end groups are alkyl esters and aromatic esters. Hydrophilic ended (also referred to as uncapped) PLGA has an end group hydrophilic in nature at the polymer terminus. PLGA with a hydrophilic end groups at the polymer terminus degrades faster than hydrophobic ended PLGA because it takes up water and undergoes hydrolysis at a faster rate (Tracy et al., Biomaterials 20:1057-1062 (1999)). The hydrophilic end groups are carboxyl, hydroxyl, and polyethylene glycol. The specific end group will typically result from the initiator employed in the polymerization process. For example, if the initiator is water or carboxylic acid, the resulting end groups will be carboxyl and hydroxyl. Similarly, if the initiator is a monofunctional alcohol, the resulting end groups will be ester or hydroxyl.

The implants formed have a ratio of hydrophilic end to hydrophobic end PLGA in the biodegradable polymer matrices of this claimed invention that range from about 10:1 to about 1:10 by weight. For example, the ratio may be 3:1, 2:1, or 1:1 by weight. In a preferred variation, an implant with a ratio of hydrophilic end to hydrophobic end PLGA of 3:1 w/w is used.

### Additional Agents

Other agents may be employed in the formulation for a variety of purposes. For example, buffering agents and preservatives may be employed. Preservatives which may be used include, but are not limited to, sodium bisulfite, sodium bisulfate, sodium thiosulfate, benzalkonium chloride, chlorobutanol, thimerosal, phenylmercuric acetate, phenylmercuric nitrate, methylparaben, polyvinyl alcohol and phenylethyl alcohol. Examples of buffering agents that may be employed include, but are not limited to, sodium carbonate, sodium borate, sodium phosphate, sodium acetate, sodium bicarbonate, and the like, as approved by the FDA for the desired route of administration. Electrolytes such as sodium chloride and potassium chloride may also be included in the formulation.

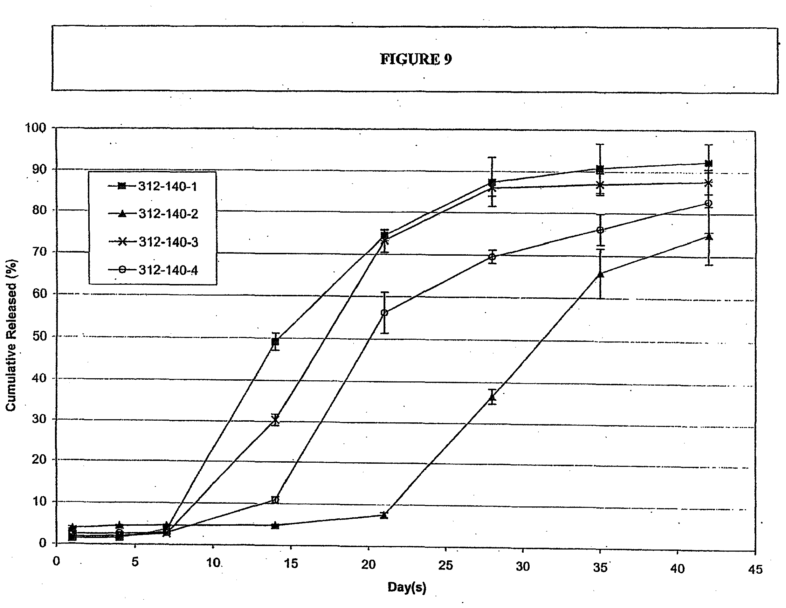

The biodegradable ocular implants are composed of biodegradable polymer matrix, wherein the biodegradable polymer matrix comprises a mixture of (i) PLGA having hydrophilic end groups, wherein said hydrophilic end group is selected from carboxyl, hydroxyl, polyethylene glycol, or a combination thereof, and (ii) PLGA having hydrophobic end groups, wherein said hydrophobic end group is an alkyl ester or aromatic ester, that accelerate or retard release of the active agent. Furthermore, the inventors believe that because hydrophilic end PLGA has a higher degradation rate than hydrophobic end PLGA due to its ability to take up water more readily, increasing the amount of hydrophilic end PLGA in the implant polymer matrix will result in faster dissolution rates. Figure 9 shows that the time from implantation to significant release of active agent (lag time) increases with decreasing amounts of hydrophilic end PLGA in the ocular implant. In Figure 9, the lag time for implants having 0% hydrophilic end PLGA (40% w/w hydrophobic end) was shown to be about 21 days. In comparison, a significant reduction in lag time was seen with implants having 10% w/w and 20% w/w hydrophilic end PLGA.

### Release Kinetics

The inventors believe the implants of the invention are formulated with particles of an active agent dispersed within a biodegradable polymer matrix, wherein the biodegradable polymer matrix comprises a mixture of (i) PLGA having hydrophilic end groups, wherein said hydrophilic end group is selected from carboxyl, hydroxyl, polyethylene glycol, or a combination thereof, and (ii) PLGA having hydrophobic end groups, wherein said hydrophobic end group is an alkyl ester or aromatic ester. The release of the active agent is achieved by erosion of the biodegradable polymer matrix and by diffusion of the particulate agent into an ocular fluid, e.g., the vitreous, with subsequent dissolution of the polymer matrix and release of the active agent. The inventors believe that the factors that influence the release kinetics include such characteristics as the size of the active agent particles, the solubility of the active agent, the ratio of active agent to polymer(s), the method of manufacture, the surface area exposed, and the erosion rate of the polymer(s). The release kinetics achieved by this form of active agent release are different than that achieved through formulations which release active agents through polymer swelling, such as with crosslinked hydrogels. In that case, the active agent is not released through polymer erosion, but through polymer swelling, which releases agent as liquid diffuses through the pathways exposed.

The inventors believe that the release rate of the active agent depends at least in part on the rate of degradation of the polymer backbone component or components making up the biodegradable polymer matrix, where in the biodegradable polymer matrix comprises a mixture of (i) PLGA having hydrophilic end groups, wherein said hydrophilic end group is selected from carboxyl, hydroxyl, polyethylene glycol, or a combination thereof, and (ii) PLGA having hydrophobic end groups, wherein said hydrophobic end group is an alkyl ester or aromatic ester. For example, condensation polymers may be degraded by hydrolysis (among other mechanisms) and therefore any change in the composition of the implant that enhances water uptake by the implant will likely increase the rate of hydrolysis, thereby increasing the rate of polymer degradation and erosion, and thus increasing the rate of active agent release.

The release kinetics of the implants of the invention are dependent in part on the surface area of the implants. A larger surface area exposes more polymer and active agent to ocular fluid, causing faster erosion of the polymer matrix and dissolution of the active agent particles in the fluid. The size and shape of the implant may also be used to control the rate of release, period of treatment, and active agent concentration at the site of implantation. At equal active agent loads, larger implants will deliver a proportionately larger dose, but depending on the surface to mass ratio, may possess a slower release rate. For implantation in an ocular region, the total weight of the implant preferably ranges, e.g., from about 100-5000 *µ*g, usually from about 500-1500 *µ*g*.* In one variation, the total weight of the implant is about 600 *µ*g*.* In another variation, the total weight of the implant is about 1200 *µ*g*.*

The bioerodible implants are typically solid, and may be formed as particles, sheets, patches, plaques, films, discs, fibers, rods, and the like, or may be of any size or shape compatible with the selected site of implantation, as long as the implants have the desired release kinetics and deliver an amount of active agent that is therapeutic for the intended medical condition of the eye. The upper limit for the implant size will be determined by factors such as the desired release kinetics, toleration for the implant at the site of implantation, size limitations on insertion, and ease of handling. For example, the vitreous chamber is able to accommodate relatively large rod-shaped implants, generally having diameters of about 0.05 mm to 3 mm and a length of about 0.5 to about 10 mm. In one variation, the rods have diameters of about 0.1 mm to about 1 mm. In another variation, the rods have diameters of about 0.3 mm to about 0.75 mm. In yet a further variation, other implants having variable geometries but approximately similar volumes may also be used.

As previously discussed, the release of an active agent from a biodegradable polymer matrix is modulated by varying the ratio of hydrophilic end PLGA to hydrophobic end PLGA in the matrix. Release rates may be further manipulated by the method used to manufacture the implant. For instance, as illustrated in Examples 4-7, extruded 60/40 w/w dexamethasone/PLGA implants having a ratio of hydrophilic end and hydrophobic end PLGA of 3:1, compared to compressed tablet implants, demonstrate a different drug release profile and concentration of agent in the vitreous over about a one month period. Overall, a lower burst of agent release and a more consistent level of agent in the vitreous is demonstrated with the extruded implants.

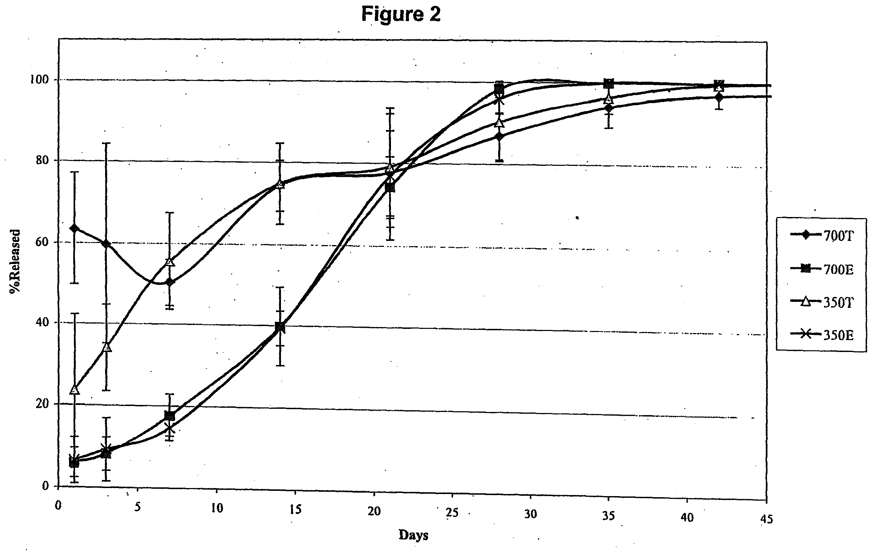

As shown in Figure 2 and Examples 4 and 5, a higher initial burst of active agent release occurs on day one after implantation with the 350 *µ*g dexamethasone compressed tablet implant (350T) in comparison to the 350 *µ*g dexamethasone extruded implant (350E). A higher initial burst of active agent release also occurs with the 700 *µ*g dexamethasone compressed implant (700T) in comparison to the 700 *µ*g dexamethasone extruded implant (700E) on day 1, as shown in Figure 2 and Examples 6 and 7.

The proportions of active agent, biodegradable polymer matrix, and any other additives may be empirically determined by formulating several implants with varying proportions and determining the release profile *in vitro* or *in vivo.* A USP approved method for dissolution or release test can be used to measure the rate of release *in vitro* (USP 24; NF 19 (2000) pp. 1941-1951). For example, a weighed sample of the implant is added to a measured volume of a solution containing 0.9% NaCl in water, where the solution volume will be such that the active agent concentration after release is less than 20% of saturation. The mixture is maintained at 37°C and stirred or shaken slowly to maintain the implants in suspension. The release of the dissolved active agent as a function of time may then be followed by various methods known in the art, such as spectrophotometrically, HPLC, mass spectroscopy, and the like, until the solution concentration becomes constant or until greater than 90% of the active agent has been released.

In one variation, the extruded implants described herewith (ratio of hydrophilic end PLGA to hydrophobic end PLGA of 3:1) may have *in vivo* cumulative percentage release profiles with the following described characteristics, as shown in Figure 2, where the release profiles are for release of the active agent *in vivo* after implantation of the implants into the vitreous of rabbit eyes. The volume of rabbit eyes is approximately 60-70% of human eyes.

At day one after implantation, the percentage *in vivo* cumulative release may be between about 0% and about 15%, and more usually between about 0% and about 10%. At day one after implantation, the percentage *in vivo* cumulative release may be less than about 15%, and more usually less than about 10%.

At day three after implantation, the percentage *in vivo* cumulative release may be between about 0% and about 20%, and more usually between about 5% and about 15%. At day three after implantation, the percentage *in vivo* cumulative release may be less than about 20%, and more usually less than about 15%.

At day seven after implantation, the percentage *in vivo* cumulative release may be between about 0% and about 35%, more usually between about 5% and about 30%, and more usually still between about 10% and about 25%. At day seven after implantation, the percentage *in vivo* cumulative release may be greater than about 2%, more usually greater than about 5%, and more usually still greater than about 10%.

At day fourteen after implantation, the percentage *in vivo* cumulative release may be between about 20% and about 60%, more usually between about 25% and about 55%, and more usually still between about 30% and about 50%. At day fourteen after implantation, the percentage *in vivo* cumulative release may be greater than about 20%, more usually greater than about 25%, and more usually still greater than about 30%.

At day twenty-one after implantation, the percentage *in vivo* cumulative release may be between about 55% and about 95%, more usually between about 60% and about 90%, and more usually still between about 65% and about 85%. At day twenty-one after implantation, the percentage *in vivo* cumulative release may be greater than about 55%, more usually greater than about 60%, and more usually still greater than about 65%.

At day twenty-eight after implantation, the percentage *in vivo* cumulative release may be between about 80% and about 100%, more usually between about 85% and about 100%, and more usually still between about 90% and about 100%. At day twenty-eight after implantation, the percentage *in vivo* cumulative release may be greater than about 80%, more usually greater than about 85%, and more usually still greater than about 90%.

At day thirty-five after implantation, the percentage *in vivo* cumulative release may be between about 95% and about 100%, and more usually between about 97% and about 100%. At day thirty-five after implantation, the percentage *in vivo* cumulative release may be greater than about 95%, and more usually greater than about 97%.

In one variation, the percentage *in vivo* cumulative release has the following characteristics: one day after implantation it is less than about 15%; three days after implantation it is less than about 20%; seven days after implantation it is greater than about 5%; fourteen days after implantation it is greater than about 25%; twenty-one days after implantation it is greater than about 60%; and twenty-eight days after implantation it is greater than about 80%. In another variation, the percentage *in vivo* cumulative release has the following characteristics: one day after implantation it is less than about 10%; three days after implantation it is less than about 15%; seven days after implantation it is greater than about 10%; fourteen days after implantation it is greater than about 30%; twenty-one days after implantation it is greater than about 65%; twenty-eight days after implantation it is greater than about 85%.

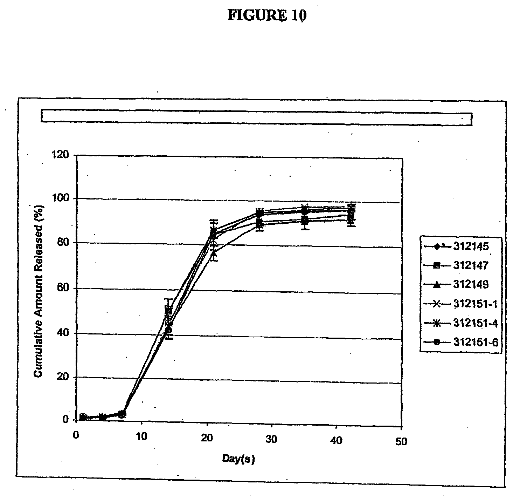

In yet another variation, the extruded implants described in this patent may have *in vitro* cumulative percentage release profiles in saline solution at 37°C with the following characteristics, as further described below, and as shown in Figure 10.

The percentage *in vitro* cumulative release at day one may be between about 0% and about 5%, and more usually between about 0% and about 3%. The percentage *in vitro* cumulative release at day one may be less than about 5%, and more usually less than about 3%.

The percentage *in vitro* cumulative release at day four may be between about 0% and about 7%, and more usually between about 0% and about 5%. The percentage *in vitro* cumulative release at day four may be less than about 7%, and more usually less than about 5%.

The percentage *in vitro* cumulative release at day seven may be between about 1% and about 10%, and more usually between about 2% and about 8%. The percentage *in vitro* cumulative release at day seven may be greater than about 1%, and more usually greater than about 2%.

The percentage *in vitro* cumulative release at day 14 may be between about 25% and about 65%, more usually between about 30% and about 60%, and more usually still between about 35% and about 55%. The percentage *in vitro* cumulative release at day 14 may be greater than about 25%, more usually greater than about 30%, and more usually still greater than about 35%.

The percentage *in vitro* cumulative release at day 21 may be between about 60% and about 100%, more usually between about 65% and about 95%, and more usually still between about 70% and about 90%. The percentage *in vitro* cumulative release at day 21 may be greater than about 60%, more usually greater than about 65%, and more usually still greater than about 70%.

The percentage *in vitro* cumulative release at day 28 may be between about 75% and about 100%, more usually between about 80% and about 100%, and more usually still between about 85% and about 95%. The percentage *in vitro* cumulative release at day 28 may be greater than about 75%, more usually greater than about 80%, and more usually still greater than about 85%.

The percentage *in vitro* cumulative release at day 35 may be between about 85% and about 100%, more usually between about 90% and about 100%, and more usually still between about 95% and about 100%. The percentage *in vitro* cumulative release at day 35 may be greater than about 85%, more usually greater than about 90%, and more usually still greater than about 95%.

In one variation, the percentage *in vitro* cumulative release has the following characteristics: after one day it is less than about 1%; after four days it is less than about 7%; after seven days it is greater than about 2%; after 14 days it is greater than about 30%; after 21 days it is greater than about 65%; after 28 days it is greater than about 80%; and after 35 days it is greater than about 90%. In another variation, the percentage *in vitro* cumulative release has the following characteristics: after one day it is less than about 3%; after four days it is less than about 5%; after seven days it is greater than about 2%; after 14 days it is greater than about 35%; after 21 days it is greater than about 70%; after 28 days it is greater than about 85%; and after 35 days it is greater than about 90%.

Besides showing a lower burst effect for the extruded implants, Figures 2 and 10 also demonstrate that after 28 days *in vivo* in rabbit eyes, or *in vitro* in a saline solution at 37°C, respectively, almost all of the active agent has been released from the implants. Furthermore, Figures 2 and 10 show that the active agent release profiles for the extruded implants *in vivo* (from the time of implantation) and *in vitro* (from the time of placement into a saline solution at 37°C) are substantially similar and follow approximately a sigmoidal curve, releasing substantially all of the active agent over 28 days. From day one to approximately day 17, the curves show approximately an upward curvature (i.e., the derivative of the curve increases as time increases), and from approximately day 17 onwards the curves show approximately a downward curvature (i.e., the derivative of the curve decreases as time increases).

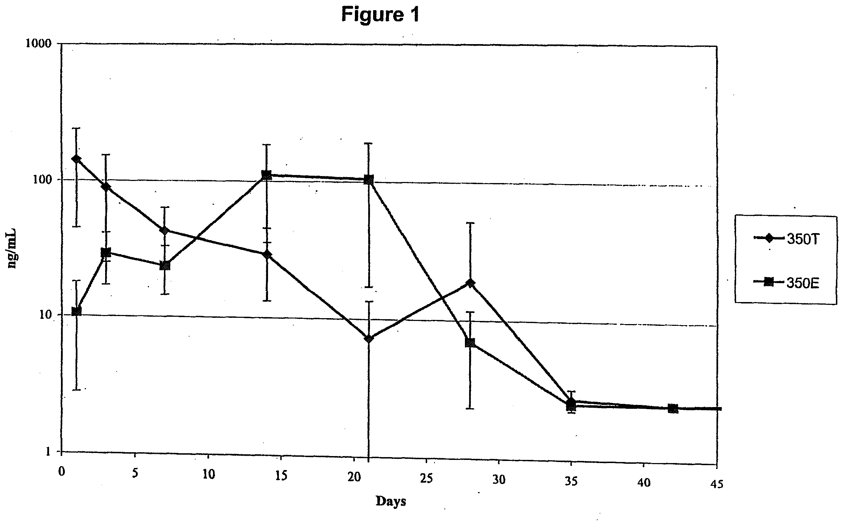

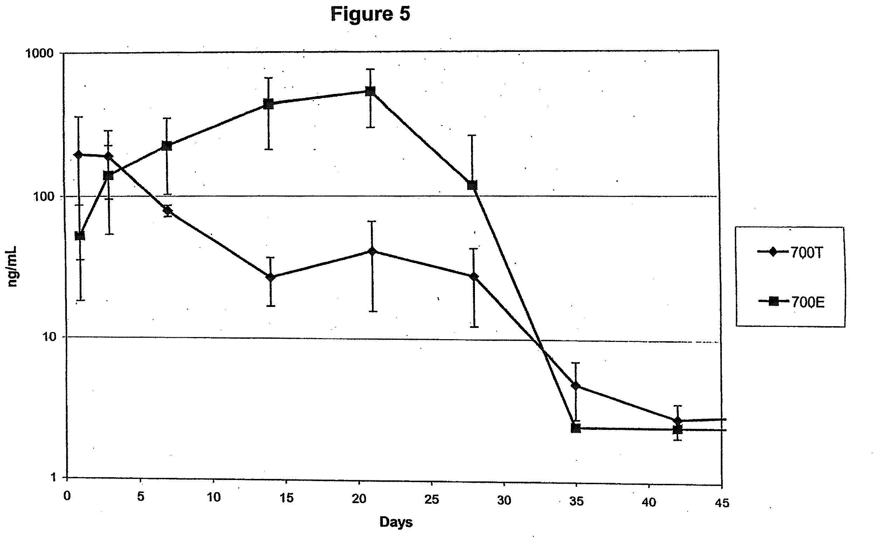

In contrast, the plots shown in Figure 2 for the 350 *µ*g and 700 *µ*g dexamethasone compressed tablet implants exhibit a higher initial burst of agent release generally followed by a gradual increase in release. Furthermore, as shown in Figures 1 and 5, implantation of a compressed implant results in different concentrations of active agent in the vitreous at various time points from implants that have been extruded. For example, as shown in Figures 1 and 5, with extruded implants there is a gradual increase, plateau, and gradual decrease in intravitreal agent concentrations. In contrast, for compressed tablet implants, there is a higher initial active agent release followed by an approximately constant decrease over time. Consequently, the intravitreal concentration curve for extruded implants results in more sustained levels of active agent in the ocular region.

In addition to the previously described implants releasing substantially all of the therapeutic agent within 35 days, by varying implant components including, but not limited to, the composition of the biodegradable polymer matrix, implants may also be formulated to release a therapeutic agent for any desirable duration of time, for example, for about one week, for about two weeks, for about three weeks, for about four weeks, for about five weeks, for about six weeks, for about seven weeks, for about eight weeks, for about nine weeks, for about ten weeks, for about eleven weeks, for about twelve weeks, or for more than 12 weeks.

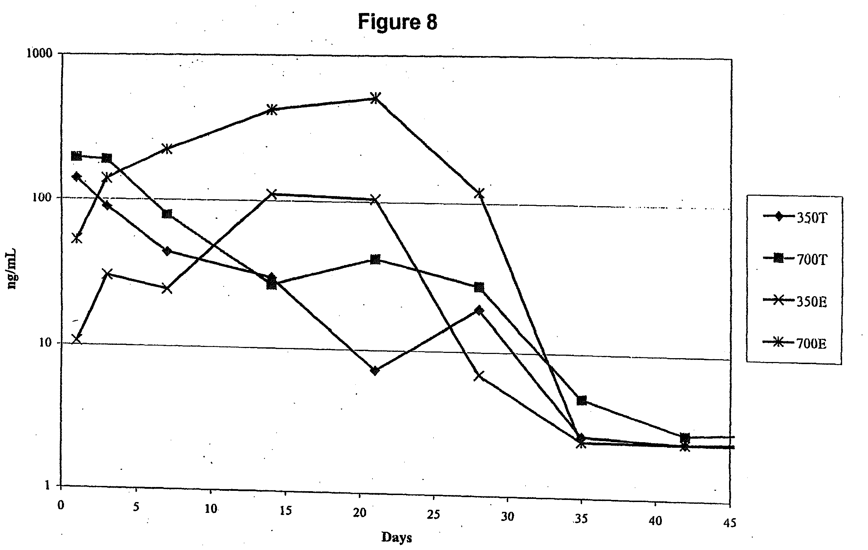

Another important feature of the extruded implants is that different concentration levels of active agent may be established in the vitreous using different doses of the active agent. As illustrated in Figure 8, the concentration of agent in the vitreous is significantly larger with the 700 *µ*g dexamethasone extruded implant than with the 350 *µ*g dexamethasone extruded implant. Different active agent concentrations are not demonstrated with the compressed tablet implant. Thus, by using an extruded implant, it is possible to more easily control the concentration of active agent in the vitreous. In particular, specific dose-response relationships may be established since the implants can be sized to deliver a predetermined amount of active agent.

### Applications

Examples of medical conditions of the eye which may be treated by the implants and methods of the invention include, but are not limited to, uveitis, macular edema, macular degeneration, retinal detachment, ocular tumors, fungal or viral infections, multifocal choroiditis, diabetic retinopathy, proliferative vitreoretinopathy (PVR), sympathetic opthalmia, Vogt Koyanagi-Harada (VKH) syndrome, histoplasmosis, uveal diffusion, and vascular occlusion. In one variation, the implants are particularly useful in treating such medical conditions as uveitis, macular edema, vascular occlusive conditions, proliferative vitreoretinopathy (PVR), and various other retinopathies.

### Method of Implantation

The biodegradable implants may be inserted into the eye by a variety of methods, including placement by forceps, by trocar, or by other types of applicators, after making an incision in the sclera. In some instances, a trocar or applicator may be used without creating an incision. In a preferred variation, a hand held applicator is used to insert one or more biodegradable implants into the eye. The hand held applicator typically comprises an 18-30 GA stainless steel needle, a lever, an actuator, and a plunger.

The method of implantation generally first involves accessing the target area within the ocular region with the needle. Once within the target area, e.g., the vitreous cavity, the lever on the hand held device is depressed to cause the actuator to drive the plunger forward. As the plunger moves forward, it pushes the implant into the target area.

### Extrusion Methods

The use of extrusion methods allows for large-scale manufacture of implants and results in implants with a homogeneous dispersion of the drug within the polymer matrix. When using extrusion methods, the polymers and active agents that are chosen are stable at temperatures required for manufacturing, usually at least about 50°C. Extrusion methods use temperatures of about 25°C to about 150°C, more preferably about 60°C to about 130°C.

Different extrusion methods may yield implants with different characteristics, including but not limited to the homogeneity of the dispersion of the active agent within the polymer matrix. Using a piston extruder, a single screw extruder, and a twin screw extruder will generally produce implants with progressively more homogeneous dispersion of the active. When using one extrusion method, extrusion parameters such as temperature, extrusion speed, die geometry, and die surface finish will have an effect on the release profile of the implants produced.

When
producing implants by extrusion methods such as using a piston extruder, a single screw extruder or a twin screw extruder, the drug and polymer are first mixed at room temperature and then heated to a temperature range of about 60°C to about 150°C, more usually to about 130°C for a time period of about 0 to about 1 hour, more usually from about 0 to about 30 minutes, more usually still from about 5 minutes to about 15 minutes, and most usually for about 10 minutes. The implants are then extruded at a temperature of about 60°C to about 130°C, preferably at a temperature of about 75°C.

In extrusion methods such as using a piston extruder, a single screw extruder or a twin screw extruder, the powder blend of active agent and PLGA is added to a single or twin screw extruder preset at a temperature of about 80°C to about 130°C, and directly extruded as a filament or rod with minimal residence time in the extruder. The extruded filament or rod is then cut into small implants having the loading dose of active agent appropriate to treat the medical condition of its intended use.

### EXAMPLES

The following examples serve to more fully describe the manner of using the above-described invention. It is understood that these examples in no way serve to limit the scope of this invention, but rather are presented for illustrative purposes.

### EXAMPLE 1 - comparative example, not falling in scope of the claims-

### Manufacture of Compressed Tablet Implants

Micronized dexamethasone (Pharmacia, Peapack, NJ) and micronized hydrophobic end 50/50 PLGA (Birmingham Polymers, Inc., Birmingham, AL) were accurately weighed and placed in a stainless steel mixing vessel. The vessel was sealed, placed on a Turbula mixer and mixed at a prescribed intensity, e.g., 96 rpm, and time, e.g., 15 minutes. The resulting powder blend was loaded one unit dose at a time into a single-cavity tablet press. The press was activated at a pre-set pressure, e.g., 25 psi, and duration, e.g., 6 seconds, and the tablet was formed and ejected from the press at room temperature. The ratio of dexamethasone to PLGA was 70/30 w/w for all compressed tablet implants.

### EXAMPLE 2

### Manufacture of Extruded Implants

Micronized dexamethasone (Pharmacia, Peapack, NJ) and unmicronized PLGA were accurately weighed and placed in a stainless steel mixing vessel. The vessel was sealed, placed on a Turbula mixer and mixed at a prescribed intensity, e.g., 96 rpm, and time, e.g., 10-15 minutes. The unmicronized PLGA composition comprised a 30/10 w/w mixture of hydrophilic end PLGA (Boehringer Ingelheim, Wallingford, CT) and hydrophobic end PLGA (Boehringer Ingelheim, Wallingford, CT). The resulting powder blend was fed into a DACA Microcompounder-Extruder (DACA, Goleta, CA) and subjected to a pre-set temperature, e.g., 115°C, and screw speed, e.g., 12 rpm. The filament was extruded into a guide mechanism and cut into exact lengths that corresponded to the designated implant weight. The ratio of dexamethasone to total PLGA (hydrophilic and hydrophobic end) was 60/40 w/w for all extruded implants.

### EXAMPLE 3

### Method for Placing Implants Into the Vitreous

Implants were placed into the posterior segment of the right eye of New Zealand White Rabbits by incising the conjunctiva and sclera between the 10 and 12 o'clock positions with a 20-gauge microvitreoretinal (MVR) blade. Fifty to 100 *µ*L of vitreous humor was removed with a 1-cc syringe fitted with a 27-gauge needle. A sterile trocar, preloaded with the appropriate implant (drug delivery system, DDS), was inserted 5 mm through the sclerotomy, and then retracted with the push wire in place, leaving the implant in the posterior segment. Sclerae and conjunctivae were than closed using a 7-0 Vicryl suture.

### EXAMPLE 4

### In vivo Release of Dexamethasone From 350µg Dexamethasone Compressed Tablet Implants

Example 4 demonstrates the high initial release but generally lower intravitreal concentration of dexamethasone from compressed tablet implants as compared to extruded implants. The 350µg compressed tablet implant (350T) was placed in the right eye of New Zealand White Rabbits as described in Example 3. Vitreous samples were taken periodically and assayed by LC/MS/MS to determine *in vivo* dexamethasone delivery performance. As seen in Figure 1, dexamethasone reached detectable mean intravitreal concentrations from day 1 (142.20 ng/ml) through day 35 (2.72 ng/ml), and the intravitreal concentration of dexamethasone gradually decreased over time.

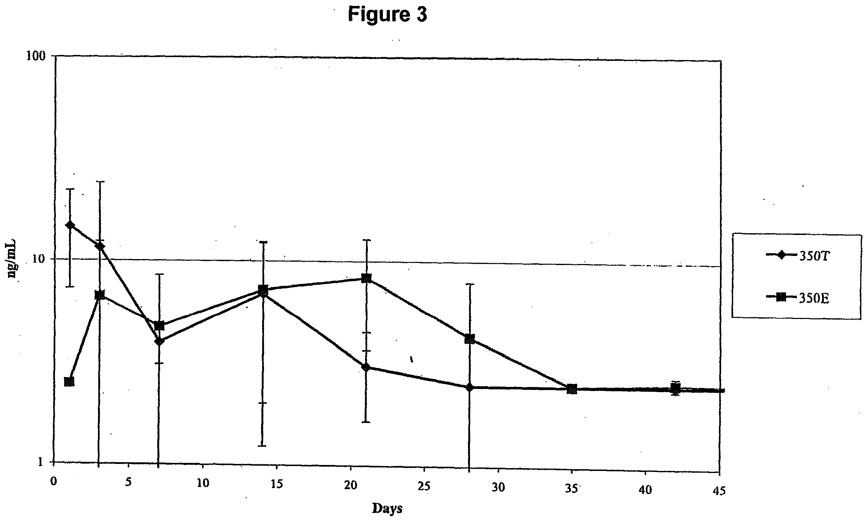

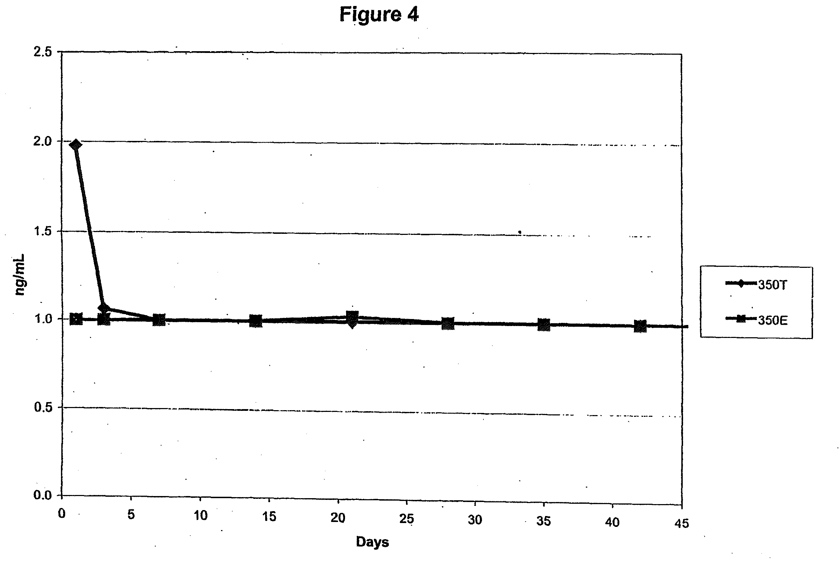

In addition to the vitreous samples, aqueous humor and plasma samples were also taken. The 350T showed a gradual decrease in aqueous humor dexamethasone concentrations over time, exhibiting a detectable mean dexamethasone aqueous humor concentration at day 1 (14.88 ng/ml) through day 21 (3.07 ng/ml), as demonstrated in Figure 3. The levels of dexamethasone in the aqueous humor strongly correlated with the levels of dexamethasone in the vitreous humor, but at a much lower level (approximately 10-fold lower). Figure 4 shows that only trace amounts of dexamethasone was found in the plasma.

### EXAMPLE 5

### In vivo Release of Dexamethasone From 350µg Dexamethasone Extruded Implants

Example 5 demonstrates the lower initial release and generally more sustained intravitreal concentration of dexamethasone from extruded implants. The 350µg extruded implant (350E) was placed in the right eye of New Zealand White Rabbits as described in Example 3. Vitreous samples were taken periodically and assayed by LC/MS/MS to determine *in vivo* dexamethasone delivery performance. Referring to Figure 1, 350E showed detectable mean vitreous humor concentrations on day 1 (10.66 ng/ml) through day 28 (6.99 ng/ml). The 350T implant had statistically significant higher dexamethasone concentrations on day 1 (p=0.037) while the 350E had a statistically significant higher dexamethasone level on day 21 (p=0.041).

In addition to the vitreous samples, aqueous humor and plasma samples were also taken. In Figure 3, the 350E showed detectable mean dexamethasone aqueous humor concentrations at day 1 (6.67 ng/ml) through day 42 (2.58 ng/ml) with the exception of day 35 in which the values were below the quantification limit. On the whole, the levels of dexamethasone in the aqueous strongly correlated with the levels of dexamethasone in the vitreous humor, but at a much lower level (approximately 10-fold lower). Figure 4 demonstrates that only a trace amount of dexamethasone was found in the plasma.

### EXAMPLE 6

### In vivo Release of Dexamethasone From 700µg Dexamethasone Compressed Tablet Implants

Example 6 also shows the high initial release and generally lower intravitreal concentration of dexamethasone from compressed tablet implants. The 700µg compressed tablet dosage form (700T) was placed in the right eye of New Zealand White Rabbits as described in Example 3. Vitreous samples were taken periodically and assayed by LC/MS/MS to determine *in vivo* dexamethasone delivery performance. As seen in Figure 5, the 700T reached detectable mean dexamethasone vitreous humor concentrations at day 1 (198.56 ng/ml) through day 42 (2.89 ng/ml), and a gradual decrease in the intravitreal dexamethasone concentration over time.

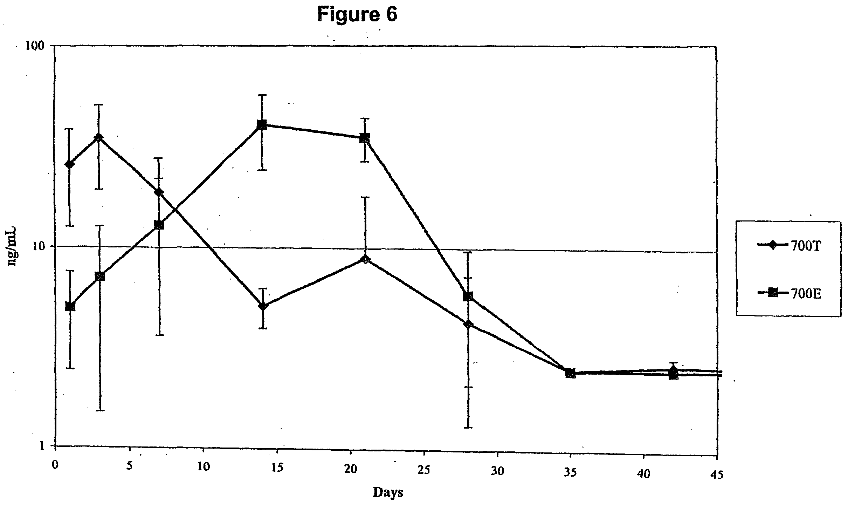

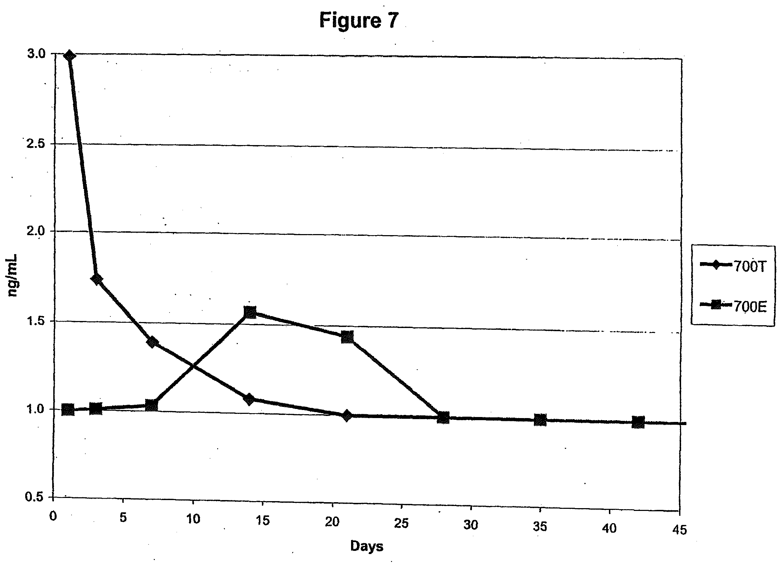

In addition to the vitreous samples, aqueous humor and plasma samples were also obtained. As seen in Figure 6, the 700T exhibited a gradual decrease in aqueous humor dexamethasone concentrations over time, and reached detectable mean dexamethasone aqueous humor concentrations at day 1 (25.90 ng/ml) through day 42 (2.64 ng/ml) with the exception of day 35 in which the values were below the quantification limit. The levels of dexamethasone in the aqueous humor strongly correlated with the levels of dexamethasone in the vitreous humor, but at a much lower level (approximately 10-fold lower). Figure 7 demonstrates that only a trace amount of dexamethasone was found in the plasma.

### EXAMPLE 7

### In vivo Release of Dexamethasone From 700µg Dexamethasone Extruded Implants

Example 7 also illustrates the lower initial release and generally higher intravitreal concentration of dexamethasone from extruded implants. The 700µg extruded implant (700E) was placed in the right eye of New Zealand White Rabbits as described in Example 3. Vitreous samples were taken periodically and assayed by LC/MS/MS to determine *in vivo* dexamethasone delivery performance. As seen in Figure 5, the 700E had a mean detectable vitreous humor concentration of dexamethasone from day 1 (52.63 ng/ml) through day 28 (119.70 ng/ml).

In addition to the vitreous samples, aqueous humor and plasma samples were also taken. As seen in Figure 6, the 700E reached a detectable mean aqueous humor concentration on day 1 (5.04 ng/ml) through day 28 (5.93 ng/ml). The levels of dexamethasone in the aqueous strongly correlated with the levels of dexamethasone in the vitreous humor, but at a much lower level (approximately 10-fold lower). Figure 7 demonstrates that only a trace amount of dexamethasone was found in the plasma.

## Claims

1. A bioerodible implant for treating a medical condition of the eye comprising an active agent dispersed within a biodegradable polymer matrix, wherein the biodegradable polymer matrix comprises a mixture of (i) PLGA having hydrophilic end groups, wherein said hydrophilic end group is selected from carboxyl, hydroxyl, polyethylene glycol, or a combination thereof, and (ii) PLGA having hydrophobic end groups, wherein said hydrophobic end group is an alkyl ester or aromatic ester, and wherein the bioerodible implant is sized for implantation in an ocular region, wherein the bioerodible implant is formed by an extrusion method, wherein the extrusion method comprises using a piston extruder, a single screw extruder or a twin screw extruder.

2. The bioerodible implant of claim 1, wherein the active agent is selected from the group consisting of ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, anti-inflammatory agents, antihypertensives, pressors, antibacterials, antivirals, antifungals, antiprotozoals, anti-infective agents, antitumor agents, antimetabolites, and antiangiogenic agents.

3. The bioerodible implant of any of the preceding claims, wherein the active agent comprises an anti-inflammatory agent or any derivative thereof.

4. The bioerodible implant of any of the preceding claims, wherein the active agent comprises a steroidal anti-inflammatory agent or any derivative thereof.

5. The bioerodible implant of claim 4, wherein the active agent is selected from the group consisting of cortisone, dexamethasone, fluocinolone, hydrocortisone, methylprednisolone, prednisolone, prednisone, triamciriolone, and any derivative thereof.

6. The bioerodible implant of claim 4, wherein the active agent comprises dexamethasone.

7. The bioerodible implant of any of the preceding claims, wherein the active agent is 10 to 90 percent by weight of the bioerodible implant.

8. The bioerodible implant of claim 7, wherein the active agent is 60 percent by weight of the bioerodible implant.

9. The bioerodible implant of any of the preceding claims, wherein the mixture has a weight ratio of hydrophilic end group PLGA to hydrophobic end group PLGA of 3:1.

10. The bioerodible implant of any of the preceding claims, wherein the ocular region is selected from the group consisting of the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina; and is preferably the vitreous cavity.

11. The bioerodible implant of any of the preceding claims for use in the treatment of a medical condition.

12. The bioerodible implant for use of claim 11, wherein the method for treating a medical condition comprises implanting into an ocular region of a subject a bioerodible implant of any one of claims 1 to 10 and delivering a therapeutic amount of an active agent to the ocular region.

13. The bioerodible implant for use of claim 12, wherein the subject is human.

14. The bioerodible implant for use any one of claims 12 or 13, wherein the medical condition of the eye is selected from the group consisting of uveitis, macular edema, macular degeneration, retinal detachment, ocular tumors, fungal infections, viral infections, multifocal choroiditis, diabetic retinopathy, proliferative vitreoretinopathy (PVR), sympathetic opthalmia, Vogt Koyanagi-Harada (VKH) syndrome, histoplasmosis, uveal diffusion, and vascular occlusion.

## Patentansprüche

1. Bioerodierbares Implantat zur Behandlung von Beschwerden am Auge, das einen Wirkstoff, der in einer biologisch abbaubaren Polymermatrix dispergiert ist, umfasst, wobei die biologisch abbaubare Polymermatrix eine Mischung aus (i) PLGA mit hydrophilen Endgruppen, wobei die hydrophile Endgruppe ausgewählt ist aus Carboxyl, Hydroxyl, Polyethylenglykol oder einer Kombination davon, und (ii) PLGA mit hydrophoben Endgruppen, wobei die hydrophobe Endgruppe ein Alkylester oder aromatischer Ester ist, umfasst und wobei das bioerodierbare Implantat für die Implantation in einen Augenbereich bemessen ist, wobei das bioerodierbare Implantat durch ein Strangpressverfahren gebildet wurde, wobei das Strangpressverfahren die Verwendung eines Kolbenextruders, eines Einschneckenextruders oder eines Doppelschneckenextruders umfasst.

2. Bioerodierbares Implantat nach Anspruch 1, wobei der Wirkstoff ausgewählt ist aus der Gruppe bestehend aus ACE-Inhibitoren, endogenen Cytokinen, Mitteln, die die Basalmembran beeinflussen, Mitteln, die das Endothelzellwachstum beeinflussen, adrenergen Agonisten oder Blockern, cholinergen Agonisten oder Blockern, Aldosereduktase-Inhibitoren, Analgetika, Anästhetika, Antiallergika, entzündungshemmenden Mitteln, Antihypertonika, Pressoren, antibakteriellen Mitteln, Virostatika, Antimykotika, Antiprotozoen, Antiinfektiva, Antitumormitteln, Antimetaboliten und Antiangiogenese-Mitteln.

3. Bioerodierbares Implantat nach mindestens einem der vorhergehenden Ansprüche, wobei der Wirkstoff ein entzündungshemmendes Mittel oder ein beliebiges Derivat davon umfasst.

4. Bioerodierbares Implantat nach mindestens einem der vorhergehenden Ansprüche, wobei der Wirkstoff ein steroidales entzündungshemmendes Mittel oder ein beliebiges Derivat davon umfasst.

5. Bioerodierbares Implantat nach Anspruch 4, wobei der Wirkstoff ausgewählt ist aus der Gruppe bestehend aus Kortison, Dexamethason, Fluocinolon, Hydrocortison, Methylprednisolon, Prednisolon, Prednison, Triamciriolon und einem beliebigen Derivat davon.

6. Bioerodierbares Implantat nach Anspruch 4, wobei der Wirkstoff Dexamethason umfasst.

7. Bioerodierbares Implantat nach mindestens einem der vorhergehenden Ansprüche, wobei der Wirkstoff 10 bis 90 Gewichtsprozent des bioerodierbaren Implantats ausmacht.

8. Bioerodierbares Implantat nach Anspruch 7, wobei der Wirkstoff 60 Gewichtsprozent des bioerodierbaren Implantats ausmacht.

9. Bioerodierbares Implantat nach mindestens einem der vorhergehenden Ansprüche, wobei die Mischung ein Gewichtsverhältnis von PLGA mit hydrophilen Endgruppen zu PLGA mit hydrophoben Endgruppen von 3:1 aufweist.

10. Bioerodierbares Implantat nach mindestens einem der vorhergehenden Ansprüche, wobei der Augenbereich ausgewählt ist aus der Gruppe bestehend aus der Vorderkammer, der Hinterkammer, dem Glaskörperraum, der Aderhaut, dem suprachoroidalen Raum, der Bindehaut, dem Subkonjunktivalraum, dem Episkleralraum, dem Intrakornealraum, dem Epikornealraum, der Lederhaut, der Pars Plana, chirurgisch induzierten avaskulären Bereichen, der Macula und der Netzhaut; und vorzugsweise der Glaskörperraum ist.

11. Bioerodierbares Implantat nach mindestens einem der vorhergehenden Ansprüche zur Verwendung bei der Behandlung von gesundheitlichen Beschwerden.

12. Bioerodierbares Implantat zur Verwendung nach Anspruch 11, wobei das Verfahren zur Behandlung von gesundheitlichen Beschwerden umfasst, dass ein bioerodierbares Implantat nach mindestens einem der Ansprüche 1 bis 10 in einen Augenbereich eines Patienten implantiert wird und eine therapeutische Menge eines Wirkstoffs dem Augenbereich zugeführt wird.

13. Bioerodierbares Implantat zur Verwendung nach Anspruch 12, wobei der Patient ein Mensch ist.

14. Bioerodierbares Implantat zur Verwendung nach Anspruch 12 oder 13, wobei die Beschwerden am Auge ausgewählt sind aus der Gruppe bestehend aus Uveitis, Makulaödem, Makuladegeneration, Netzhautablösung, Augentumoren, Pilzinfektionen, Virusinfektionen, multifokaler Choroiditis, diabetischer Retinopathie, proliferativer Vitreoretinopathie (PVR), sympathischer Ophthalmie, Vogt Koyanagi-Harada (VKH) - Syndrom, Histoplasmose, Uvealdiffusion und Gefäßverschluss.

## Revendications

1. Implant bioérodable pour le traitement d'une affection médicale de l'oeil, comprenant un agent actif dispersé dans une matrice polymère biodégradable, dans lequel la matrice polymère biodégradable comprend un mélange de (i) PLGA ayant des groupes terminaux hydrophiles, dans lequel ledit groupe terminal hydrophile est sélectionné parmi un carboxyle, un hydroxyle, un polyéthylène glycol ou une combinaison de ceux-ci, et de (ii) PLGA ayant des groupes terminaux hydrophobes, dans lequel ledit groupe terminal hydrophobe est un ester d'alkyle ou un ester aromatique, et dans lequel l'implant bioérodable est dimensionné pour une implantation dans une région oculaire, dans lequel l'implant bioérodable est formé par un procédé d'extrusion, dans lequel le procédé d'extrusion comprend l'utilisation d'une extrudeuse à piston, d'une extrudeuse à vis unique ou d'une extrudeuse à double vis.

2. Implant bioérodable selon la revendication 1, dans lequel l'agent actif est sélectionné dans le groupe constitué des inhibiteurs ace, des cytokines endogènes, des agents qui influencent la membrane basale, des agents qui influencent la croissance des cellules endothéliales, des agonistes ou inhibiteurs adrénergiques, des agonistes ou inhibiteurs cholinergiques, des inhibiteurs d'aldose réductase, des analgésiques, des anesthésiques, des antiallergiques, des agents anti-inflammatoires, des antihypertensifs, des vasopresseurs, des antibactériens, des antiviraux, des antifongiques, des antiprotozoaires, des agents anti-infectieux, des agents anti-tumoraux, des antimétabolites et des agents antiangiogéniques.

3. Implant bioérodable selon l'une quelconque des revendications précédentes, dans lequel l'agent actif comprend un agent anti-inflammatoire ou un dérivé quelconque de celui-ci.

4. Implant bioérodable selon l'une quelconque des revendications précédentes, dans lequel l'agent actif comprend un agent anti-inflammatoire stéroïdien ou un dérivé quelconque de celui-ci.

5. Implant bioérodable selon la revendication 4, dans lequel l'agent actif est sélectionné dans le groupe constitué de la cortisone, de la dexaméthasone, de la fluocinolone, de l'hydrocortisone, de la méthylprednisolone, de la prednisolone, de la prednisone, de la triamciriolone et d'un quelconque de leurs dérivés.

6. Implant bioérodable selon la revendication 4, dans lequel l'agent actif comprend la dexaméthasone.

7. Implant bioérodable selon l'une quelconque des revendications précédentes, dans lequel l'agent actif constitue 10 à 90 pour cent en poids de l'implant bioérodable.

8. Implant bioérodable selon la revendication 7, dans lequel l'agent actif constitue 60 pour cent en poids de l'implant bioérodable.

9. Implant bioérodable selon l'une quelconque des revendications précédentes, dans lequel le mélange a un rapport pondéral du groupe terminal hydrophile PLGA au groupe terminal hydrophobe PLGA de 3:1.

10. Implant bioérodable selon l'une quelconque des revendication précédentes, dans lequel la région oculaire est sélectionnée dans le groupe constitué de la chambre intérieure, de la chambre postérieure, de la cavité vitreuse, de la choroïde, de l'espace suprachoroïdien, de la conjonctive, de l'espace sous-conjonctival, de l'espace épiscléral, de l'espace intracornéen, de l'espace épicornéen, de la sclérotique, de la zone postérieure, des régions avasculaires chirurgicalement induites, de la macule et de la rétine ; et est de préférence la cavité vitreuse.

11. Implant bioérodable selon l'une quelconque des revendications précédentes pour utilisation dans le traitement d'un état médical.

12. Implant bioérodable pour utilisation selon la revendication 11, dans lequel le procédé pour traiter un état médical comprend l'implantation dans une région oculaire d'un sujet d'un implant bioérodable selon l'une quelconque des revendications 1 à 10 et la fourniture d'une quantité thérapeutique d'un agent actif à la région oculaire.

13. Implant bioérodable pour utilisation selon la revendication 12, dans lequel le sujet est humain.

14. Implant bioérodable pour utilisation selon l'une quelconque des revendications 12 ou 13, dans lequel l'affection médicale de l'oeil est sélectionnée dans le groupe constitué d'une uvéite, d'un oedème maculaire, d'une dégénérescence maculaire, d'un décollement de la rétine, de tumeurs oculaires, d'infections fongiques, d'infections virales, d'une choroïdite multifocale, d'une rétinopathie diabétique, d'une vitréorétinopathie proliférative (VRP), d'une ophtalmie sympathique, du syndrome de Vogt Koyanagi-Harada (VKH), d'une histoplasmose, d'une diffusion de l'uvée et d'une occlusion vasculaire.
